# EUROPEAN PATENT APPLICATION

(11) **EP 2 853 256 A1**
(43) Date of publication of application: **01.04.2015**
(21) Application number: 13185928.2
(22) Date of filing: 25.09.2013
(51) Int. Cl.: A61K 8/64, A61Q 11/00, A61Q 11/02, A61K 8/02, A61K 8/19

(54) **Dental care product for tooth whitening**

(71) Applicant: Credentis AG, 5210 Windisch (CH)
(72) Inventor: Kunzelmann, Karl-Heinz, 85435 Erding (DE); Lysek, Dominikus Amadeus, 5210 Windisch (CH); Hug, Michael, 4800 Zofingen (CH)
(74) Representative: Moré, Solveig Helga

(57) **Abstract**

The present invention relates to the field of dental care, in particular, to a dental care product such as toothpaste, tooth gel, cream or dentifrice for whitening teeth, wherein the product comprises mineral particles such as crystals and a compound, preferably, a protein capable of forming a matrix which is a hydrogel, wherein the product comprises a fluorophore. The mineral particles may comprise, e.g., calcium phosphate, preferably, hydroxyapatite, preferably, in crystalline form. The protein matrix may comprise, e.g., a self-assembling peptide. The product also comprises a fluorophore, which may be a fluorescent amino acid residue of the protein matrix. The invention also relates to cosmetic use of the dental care product for whitening teeth or for use in treatment of a sensitive tooth or sensitive teeth and/or in prevention or treatment of caries, as well as a related method for tooth whitening.

SEQUENCE LISTING

<110>  credentis ag

<120>  Dental care product for tooth whitening

<130>  P91271

<160>  19

<170>  PatentIn version 3.5

<210>  1
<211>  11
<212>  PRT
<213>  Artificial Sequence

<220>
<223>  self-assembling peptide

<400>  1

Gln Gln Arg Phe Glu Trp Glu Phe Glu Gln Gln
1               5                   10


<210>  2
<211>  11
<212>  PRT
<213>  Artificial Sequence

<220>
<223>  self-assembling peptide


<220>
<221>  MOD_RES
<222>  (3)..(3)
<223>  Orn

<220>
<221>  MOD_RES
<222>  (5)..(5)
<223>  Orn

<220>
<221>  MOD_RES
<222>  (7)..(7)
<223>  Orn

<400>  2

Gln Gln Xaa Phe Xaa Trp Xaa Phe Gln Gln Gln
1               5                   10


<210>  3
<211>  11
<212>  PRT
<213>  Artificial Sequence

<220>
<223>  self-assembling peptide

<220>
<221>  MOD_RES
<222>  (5)..(5)
<223>  Orn

<220>
<221>  MOD_RES
<222>  (7)..(7)
<223>  Orn

<400>  3

Gln Gln Arg Phe Xaa Trp Xaa Phe Glu Gln Gln
1               5                   10


<210>  4
<211>  11
<212>  PRT
<213>  Artificial Sequence

<220>
<223>  self-assembling peptide

<400>  4

Gln Gln Arg Phe Gln Trp Gln Phe Glu Gln Gln
1               5                   10


<210>  5
<211>  11
<212>  PRT
<213>  Artificial Sequence

<220>
<223>  self-assembling peptide

<400>  5

Gln Gln Glu Phe Glu Trp Glu Phe Glu Gln Gln
1               5                   10


<210>  6
<211>  10
<212>  PRT
<213>  Artificial Sequence

<220>
<223>  self-assembling peptide


<220>
<221>  MOD_RES
<222>  (3)..(3)
<223>  Orn

<220>
<221>  MOD_RES
<222>  (5)..(5)

<223>  Orn

<220>
<221>  MOD_RES
<222>  (7)..(7)
<223>  Orn

<220>
<221>  MOD_RES
<222>  (9)..(9)
<223>  Orn

<400>  6

Gln Gln Xaa Phe Xaa Trp Xaa Phe Xaa Gln
1               5                   10


<210>  7
<211>  11
<212>  PRT
<213>  Artificial Sequence

<220>
<223>  self-assembling peptide

<400>  7

Glu Gln Glu Phe Glu Trp Glu Phe Glu Gln Glu
1               5                   10


<210>  8
<211>  11
<212>  PRT
<213>  Artificial Sequence

<220>
<223>  self-assembling peptide

<400>  8

Gln Gln Glu Phe Glu Trp Glu Phe Glu Gln Gln
1               5                   10


<210>  9
<211>  11
<212>  PRT
<213>  Artificial Sequence

<220>
<223>  self-assembling peptide

<400>  9

Glu Ser Glu Phe Glu Trp Glu Phe Glu Ser Glu
1               5                   10


<210>  10
<211>  11
<212>  PRT

<213>  Artificial Sequence

<220>
<223>  self-assembling peptide


<220>
<221>  MOD_RES
<222>  (3)..(3)
<223>  Orn

<220>
<221>  MOD_RES
<222>  (5)..(5)
<223>  Orn

<220>
<221>  MOD_RES
<222>  (7)..(7)
<223>  Orn

<220>
<221>  MOD_RES
<222>  (9)..(9)
<223>  Orn

<400>  10

Gln Gln Xaa Phe Xaa Trp Xaa Phe Xaa Gln Gln
1               5                   10


<210>  11
<211>  11
<212>  PRT
<213>  Artificial Sequence

<220>
<223>  self-assembling peptide


<220>
<221>  MOD_RES
<222>  (1)..(1)
<223>  Orn

<220>
<221>  MOD_RES
<222>  (3)..(3)
<223>  Orn

<220>
<221>  MOD_RES
<222>  (5)..(5)
<223>  Orn

<220>
<221>  MOD_RES
<222>  (7)..(7)
<223>  Orn

<220>
<221>  MOD_RES

<222>  (9)..(9)
<223>  Orn

<220>
<221>  MOD_RES
<222>  (11)..(11)
<223>  Orn

<400>  11

Xaa Gln Xaa Phe Xaa Trp Xaa Phe Xaa Gln Xaa
1               5                   10


<210>  12
<211>  11
<212>  PRT
<213>  Artificial Sequence

<220>
<223>  self-assembling peptide


<220>
<221>  MOD_RES
<222>  (3)..(3)
<223>  Orn

<220>
<221>  MOD_RES
<222>  (5)..(5)
<223>  Orn

<220>
<221>  MOD_RES
<222>  (7)..(7)
<223>  Orn

<220>
<221>  MOD_RES
<222>  (9)..(9)
<223>  Orn

<400>  12

Ser Ser Xaa Phe Xaa Trp Xaa Phe Xaa Ser Ser
1               5                   10


<210>  13
<211>  11
<212>  PRT
<213>  Artificial Sequence

<220>
<223>  self-assembling peptide

<400>  13

Ser Ser Arg Phe Glu Trp Glu Phe Glu Ser Ser
1               5                   10

<210>  14
<211>  11
<212>  PRT
<213>  Artificial Sequence

<220>
<223>  self-assembling peptide


<220>
<221>  MOD_RES
<222>  (5)..(5)
<223>  Orn

<220>
<221>  MOD_RES
<222>  (7)..(7)
<223>  Orn

<400>  14

Ser Ser Arg Phe Xaa Trp Xaa Phe Glu Ser Ser
1               5                   10


<210>  15
<211>  11
<212>  PRT
<213>  Artificial Sequence

<220>
<223>  self-assembling peptide


<220>
<221>  MOD_RES
<222>  (3)..(3)
<223>  Orn

<220>
<221>  MOD_RES
<222>  (5)..(5)
<223>  Orn

<220>
<221>  MOD_RES
<222>  (7)..(7)
<223>  Orn

<220>
<221>  MOD_RES
<222>  (9)..(9)
<223>  Orn

<400>  15

Gln Gln Xaa Phe Xaa Trp Xaa Phe Xaa Gln Gln
1               5                   10


<210>  16
<211>  11
<212>  PRT

<213>  Artificial Sequence

<220>
<223>  self-assembling peptide

<400>  16

Asn Asn Arg Phe Glu Trp Glu Phe Glu Asn Asn
1               5                   10


<210>  17
<211>  11
<212>  PRT
<213>  Artificial Sequence

<220>
<223>  self-assembling peptide


<220>
<221>  MOD_RES
<222>  (5)..(5)
<223>  Orn

<220>
<221>  MOD_RES
<222>  (7)..(7)
<223>  Orn

<400>  17

Asn Asn Arg Phe Xaa Trp Xaa Phe Glu Asn Asn
1               5                   10


<210>  18
<211>  11
<212>  PRT
<213>  Artificial Sequence

<220>
<223>  self-assembling peptide

<400>  18

Thr Thr Arg Phe Glu Trp Glu Phe Glu Thr Thr
1               5                   10


<210>  19
<211>  11
<212>  PRT
<213>  Artificial Sequence

<220>
<223>  self-assembling peptide


<220>
<221>  MOD_RES
<222>  (5)..(5)
<223>  Orn

<220>
<221>  MOD_RES
<222>  (7)..(7)
<223>  Orn

<400>  19

Thr Thr Arg Phe Xaa Trp Xaa Phe Glu Thr Thr
1               5                   10

## Description

The present invention relates to the field of dental care, in particular, to a dental care product such as toothpaste, tooth gel, cream or dentifrice for whitening teeth, wherein the product comprises mineral particles such as crystals and a compound, preferably, a protein capable of forming a matrix which is a hydrogel, wherein the product comprises a fluorophore. The mineral particles may comprise, e.g., calcium phosphate, preferably, hydroxyapatite, preferably, in crystalline form. The protein matrix may comprise, e.g., a self-assembling peptide. The product also comprises a fluorophore, which may be a fluorescent amino acid residue of the protein matrix. The invention also relates to cosmetic use of the dental care product for whitening teeth or for use in treatment of a sensitive tooth or sensitive teeth and/or in prevention or treatment of caries, as well as a related method for tooth whitening.

Enamel of teeth is the hardest substance of the human body. It is composed of about 98% hydroxyapatite, a crystalline form of calcium phosphate, and some organic components. A thin layer of enamel forms on the surface of teeth with dentin as a base. Enamel is tougher and suitable for absorbing the stress of mastication without fracture. Dentin also comprises hydroxyapatite, with a higher porosity, and a higher content of organic structure.

Both extrinsic and intrinsic reasons contribute to discoloration of teeth. For example, coffee, tea, wine, carrots, oranges or tobacco can leave stains on the enamel surface. Certain antibiotics, excessive fluoride uptake or hereditary diseases can cause intrinsic discoloration. Tooth discoloration can be an important aesthetic problem for dental patients. For example, in the UK, around 20% of people are dissatisfied with their teeth colour. In the USA, 34 % seem to be dissatisfied.

Often, superficial stains can be removed by thorough cleaning of teeth by the patient or a health professional. Polishing with abrasive material is sometimes used to this end, for example employing pastes which comprise particles of calcium phosphate, chalk, pumice or silica. If the patient desires a further more intrinsic reduction in teeth discoloration, chemical bleaching is the classical option. Various bleaching techniques are known, which are based upon an oxidizing agent such as peroxide. Bleaching can be performed by a health professional in a dental clinic or by the patient at home. For this, prescription products for overnight bleaching or bleaching toothpastes can be used.

However, in recent years, awareness of side effects associated with bleaching, such as demineralisation, erosion and tooth sensitivity caused by peroxides, has increased (e.g., Dahl et al., 2003). It has been suggested to use hydroxyapatite nanocrystals to remineralize tooth surfaces damaged e.g., by bleaching (Mohd et al., 2007, Jiang et al., 2008, Lim et al., 2009, Roveri et al., 2009). Hydroxyapatite crystalline particles, which closely mimic the natural material of the tooth, can be deposited on the tooth enamel. In addition to filling in scratches or eroded parts of a tooth, and prevention or treatment of caries, deposited hydroxyapatite can counteract hypersensitivity of teeth caused by exposition of dentin tubule upon recession of gums (WO 2007/137606 A1).

Amorphous Calcium Phosphate stabilised with phosphoproteins such as caseinphosphopeptide (CPP-ACP) has been used in oral care products for preventing and treating caries lesions (z.B. US 20050037948 A1, US 20080075675 A1, US 20100297203 A1).

It was found that calcium phosphate such as hydroxyapatite in particulate form also has whitening properties independent of bleaching or polishing (Niwa et al., 2001, Dabanoglu et al, 2009). Dabanoglu et al. compared different materials, e.g., nano-hydroxyapatite or nano-tricalcium phosphate or a dissolvable polymer film (methacrylic acid-ethyl acrylate copolymers) comprising nano-hydroxyapatite with regard to their whitening properties. They achieved a colour change, measured spectrometically as ΔE (L*a*b scale), with all tested materials. Measurement can be performed according to ISO 28399. The effect increased with three applications to a ΔE of about 3, which decreased with some materials after subjecting the treated teeth to shear force. It is noted that the average casual viewer can notice the difference between two colours that are 3-4 ΔE apart. A trained eye can differentiate between two colours that are 2-3 ΔE apart. Thus, while a perceivable change could be generated, there is still room for improvement.

WO 2013/068020 discloses a dental care product comprising hydroxyapatite having a surface functionalized with lactoferrin. This is suggested to form a thin film on the enamel surface, which improves teeth remineralisation and has an antibacterial effect.

US 20100247589 A1 describes an oral care system with different components such as a monomeric peptide with a part binding to oral surfaces and a second binding element and a composition which may comprise particles which may bind to the second binding element and which comprise a benefit agent such as a whitening agent, e.g., TiO₂ or hydroxyapatite particles, or an anti-stain agent or enzyme. The peptide is suggested to facilitate binding of the benefit agent to the oral surface.

Raoufi et al., 2010, have compared a commercial over the counter calcium peroxide toothpaste and a hydroxyapatite toothpaste (intended for bleaching or whitening teeth, respectively) with a fluoride placebo toothpaste, and found no objective whitening effect for any of the toothpastes in a 12 week clinical trial.

There is a need in the art for a dental care product effective in tooth whitening, which preferably minimizes disadvantages of bleaching such as demineralisation and tooth sensitivity, which is safe, preferably, for commercial over the counter sale, and which can be administered by the patient or consumer.

This problem was addressed by the present inventors. The invention provides a dental care product and its uses as described in the claims. In particular, the invention provides a dental care product comprising 0.5-60 wt%, preferably, 0.5-40 wt%, 1-30 wt %, 5-20 wt% or 10-15 wt% mineral particles, the particles having a size of 1-1200 nm, and 0.01-10 wt%, preferably, 0.1-5 wt%, 0.2-2 wt%, 0.5-1 wt% of an organic compound capable of forming an organic matrix, preferably, a protein capable of forming a protein matrix. The protein matrix is a hydrogel. The dental care product comprises a fluorophore.

The inventors surprisingly found that, by mixing mineral particles with a suitable organic matrix such as a suitable protein matrix in a dental care product according to the invention, the previously described whitening effect of hydroxyapatite particles on teeth can be significantly increased. In particular, the invention provides a dental care product capable of producing a difference in whiteness of a tooth, measured on the L*a*b scale, of ΔE of more than 5 after 3 applications.

In the context of the present invention, the protein is capable of forming a protein matrix or hydrogel, in particular, through self-assembly. Preferably, the protein matrix is present in the dental care product in the form of a hydrogel, i.e. the proteins are not present as monomers. The term protein in the context of the invention relates to a protein comprising more than 100 amino acids and/or a peptide having 7-100 amino acids. The protein may comprise natural and/or non-natural amino acid residues such as ornithine. Preferably, the protein of the invention is selected from the group comprising amelogenin, serum albumin (preferably, bovine serum albumin or human serum albumin), lysozyme, a self-assembling peptide, a supramolecular assembly, and members of the collagen protein family (preferably, type I collagen, in particular, human or bovine collagen), e.g., in the form of gelatine, or other proteins rich in aromatic-residues (e.g., comprising more than 10% or more than 20% of aromatic residues). All of these proteins are known to be capable of forming a hydrogel.

The inventors have shown that a matrix of said protein and the mineral particles on the tooth surface can be formed, which has an increased whitening effect compared to the layer of mineral particles deposited on the tooth surface with previous methods (e.g., Dabanoglu et al, 2009). The structures incorporating the mineral particles of the present invention are believed to be preformed in the dental care product and stable under oral conditions. They are also at least partly resistant to brushing with an ultrasound toothbrush.

Self-assembling peptides are provided, e.g., in WO 2004/007532 A1, which is fully incorporated herein by reference. WO 2004/007532 A1 discloses peptides that are capable of forming three-dimensional scaffolds, thereby promoting nucleation of de-novo calcium phosphate. These artificial peptides assemble in one dimension to form beta-sheet, and higher order assemblies such as tape-like assemblies. Three-dimensional supramolecular structures of self-assembling proteins can be formed, which have an affinity for calcium phosphate.

Several other self-assembling peptides (SAP) which may be employed have been described in the prior art. For example, WO 2010/041636 A1 describes a bioadsorbable peptide tissue occluding agent containing an artificial peptide having 8-200 amino acid residues with the hydrophilic amino acids and hydrophobic amino acids alternately bonded, which self-assembles into a beta-structure at physiological pH. Self-assembling peptides with alternating hydrophobic and hydrophilic residues or stretches which interact with the extracellular matrix are also disclosed in WO 2008/113030 A2. WO 2010/103887 A1 discloses self-assembling peptides, which comprise basic, hydrophobic and acidic amino acids of a specific primary sequence and peptide gels thereof which have high strength.

Another application, WO 2007/000979 A1, describes self-assembling peptides with polar and non-polar amino acids. The peptides are capable of forming a beta-sheet structure in which the non-polar amino acid residues are arranged on one side of the structure in the assembled form. Amphiphilic self-assembling peptides for use as stable macroscopic membranes, which are used in biomaterial applications, such as slow-diffusion drug delivery, are described in US 6,548,630.

EP 2 327 428 A2 refers to a pharmaceutical composition comprising self-assembling peptide nanofibers, which are complementary to each other, and at least one cell for repairing damaged tissue, such as tissue after a myocardial infarction.

In the context of the present invention, self-assembling peptides taught in WO 2004/007532 A1 are specifically preferred. Most preferably, said protein is the self-assembling peptide designated oligopeptide 104 (SEQ ID NO: 1, QQRFEWEFEQQ) or the self-assembling peptide having SEQ ID NO: 2, QQOFOWOFQQQ, or it comprises said peptide. It may also be a self-assembling peptide having at least 60% sequence identity to a peptide consisting of SEQ ID NO: 1, 2, 3, 4, 5, 6, 7, 8, 9, 10, 11, 12, 13, 14, 15, 16, 17, 18 or 19. Preferably, the peptide has at least 70%, at least 80%, or at least 90% sequence identity to a peptide consisting of SEQ IDs, preferably, SEQ ID NO: 1 or SEQ ID NO: 2. Most preferably, the peptide has at least 60%, at least 70%, at least 80%, or at least 90% sequence identity to a peptide consisting of SEQ ID NO: 1 or is said peptide. Alternatively, the peptide may have at least 60%, at least 70%, at least 80%, or at least 90% sequence identity to a peptide consisting of SEQ ID NO: 2 or be said peptide. Examples of self-assembling peptides that may be employed in the invention are provided in Table 1 below. Self assembling peptides may be modified peptides, comprising an Ac-N-terminus and/or NH2-C-Terminus, or non-modified peptides.

**Table 1:**

| **SEQ ID NO** | **sequence** |
|---|---|
| SEQ ID NO: 1 | QQRFEWEFEQQ |
| SEQ ID NO: 2 | QQOFOWOFQQQ |
| SEQ ID NO: 3 | QQRFOWOFEQQ |
| SEQ ID NO: 4 | QQRFQWQFEQQ |
| SEQ ID NO: 5 | QQEFEWEFEQQ |
| SEQ ID NO: 6 | QQOFOWOFOQ |
| SEQ ID NO: 7 | EQEFEWEFEQE |
| SEQ ID NO: 8 | QQEFEWEFEQQ |
| SEQ ID NO: 9 | ESEFEWEFESE |
| SEQ ID NO: 10 | QQOFOWOFOQQ |
| SEQ ID NO: 11 | OQOFOWOFOQO |
| SEQ ID NO: 12 | SSOFOWOFOSS |
| SEQ ID NO: 13 | SSRFEWEFESS |
| SEQ ID NO: 14 | SSRFOWOFESS |
| SEQ ID NO: 15 | QQOFOWOFOQQ |
| SEQ ID NO: 16 | NNRFEWEFENN |
| SEQ ID NO: 17 | NNRFOWOFENN |
| SEQ ID NO: 18 | TTRFEWEFETT |
| SEQ ID NO: 19 | TTRFOWOFETT |

To be able to bind the mineral particles on a tooth surface, the matrix has to be able to bind the mineral particles and adhere to the tooth surface. The matrix thus comprises binding sites for the mineral particles which enable it to bind the calcium particles on the tooth surface. For example, charged amino acid residues such as Glu or Orn on the surface of self-assembling peptides bind to hydroxyapatite particles and to the tooth surface, which is also substantially formed of hydroxyapatite. Without intending to be bound by the theory, it is believed that both reactions increase the stability of the formed complex to generate a more permanent whitening effect. A capability for three-dimensional self-organization, which is e.g., found in collagen, supramolecular assemblies or in self-assembling peptides, is important for binding. In general, highly charged surfaces will promote adhesion of the mineral particles. The protein-matrices work particularly well when their surface shows glutamate or ornithine residues which may attach to calcium phosphate or to other mineral particles. Preferably, the protein comprises 5% or more, 10% or more, 20% or more or 30% or more charged amino acid residues, such as glutamate and/or ornithine residues.

The dental care product comprises a fluorophore. Trp is a preferred fluorophore. Preferably, said fluorophore is an amino acid residue of the protein matrix, preferably Trp, Tyr and/or Phe. Preferably 5% or more, 10% or more, 20% or more or 30% or more of the residues of the matrix protein are Trp, Tyr and/or Phe. Most preferably, 5% or more, 10% or more, 20% or more or 30% or more of the residues of the matrix protein are Trp.

In one embodiment, the protein comprises 5% or more, 10% or more, 20% or more or 30% or more charged amino acid residues such as glutamate and/or ornithine, and the protein comprises 5% or more, 10% or more, 20% or more or 30% or more fluorescent amino acid residues such as Trp.

In an alternative embodiment, the dental care product comprises a fluorophore that is no amino acid residue of the protein, and which in one embodiment is not covalently bound to the matrix. Covalent linkage of a fluorophore to the protein is also envisioned. For example, the fluorophore may be a derivative of phthalocyanine, e.g., copper phthalocyanine (covarin blue). Embedding such a fluorophore into the combination of matrix and mineral particles as described above surprisingly leads to a more permanent and more intense whitening effect compared to incorporation of such fluorophores into a conventional toothpaste. Of course, the dental care product may also comprise both a fluorophore that is an amino acid residue of the protein and an additional fluorophore that is not. However, surprisingly, addition of a fluorophore to a dental care product of the invention is not required to achieve the whitening effect, if the protein comprises a fluorescent amino acid residue as described above.

The mineral particles preferably comprise calcium phosphate or consist thereof. Calcium phosphate may, in the context of the present invention, be monocalciumphosphat-monohydrate (MCPM) Ca(H₂PO4)₂*H₂O, monocalciumphosphate anhydrate (MCPA) Ca(H₂PO4)₂, dicalciumphosphate dihydrate (DCPD, Brushit), CaHPO₄*2H₂O, dicalciumphosphate anhydrate (DCPA, Monetit) CaHPO_{4],} Octacalciumphosphate (OCP) Cag(HPO₄)₂(PO₄)₄ * 5H₂O, α-tricalciumphosphate (α-TCP) α-Ca₃(PO₄)₂, β-tricalciumphosphate β -TCP) β -Ca₃(PO₄)₂, amorphous calcium phosphate (ACP) Caₓ(PO₄)y * nH₂O, calcium-deficient hydroxyapatite (CDHA) Ca₁₀-x(HPO₄)ₓ(PO₄)₆-x(OH)₂₋ₓ (0<x<1), hydroxyapatite (HA) Ca₁₀(PO₄)₆(OH)₂, or tetracalcium-phosphate (TTCP) Ca₄(PO₄)₂O) or a mixture of different calcium phosphates. In one embodiment, the particles or have a degree of crystallinity of 40% or more, e.g., 40-60%, 60% or more, 80% or more or 90% or more, or they are crystals. A higher degree of crystallinity is expected to make the effect on whiteness of teeth more long-lasting. Throughout the invention, the calcium phosphate preferably is hydroxyapatite. The hydroxyapatite may be substituted hydroxyapatite e.g., carbonate hydroxyapatite and zinc carbonate hydroxyapatite, or pure calcium phosphate, preferably, in crystalline form. In the context of the invention, reference to calcium phosphate or hydroxyapatite includes reference to derivatised calcium phosphates or hydroxyapatites of this kind unless otherwise mentioned. Of course, the calcium phosphate or hydroxyapatite may also consist of CaPO₄ (and of course crystal water as appropriate for the respective crystal form) only. The mineral may also be a bioglass (comprising acid soluble Calcium silicates), Kaolin (Al₂Si₂O₅(OH)₄) or TiO₂ in its different crystal forms.

The hydroxyapatite particles may be obtained according to methods disclosed, e.g., in Roveri, Battistelli et al., 2009, EP 1 762 215 A1, US 20050037948 A1, US 20080075675 A1, US 20100247589 A1, US 20100297203 A1, WO 2007/137606 A1, or WO 2013/068020 A1. Preferably, the hydroxyapatite is obtainable according to WO 2007/137606 A1 and can be commercially obtained from Budenheim, Budenheim, Germany.

The size of the mineral particles preferably is measured by granulometry, e.g., with a light scattering particle size distribution analizer (such as LA-950, Horiba, Kyoto, Japan). The form of the crystals preferably is needle-shaped, but it may also be rod-shaped or acicular. The size of the particles is 10-1200 nm, i.e., 90% of particles, preferably, 95% or 100% or particles have this size.

In one embodiment, 30-100 % or 50-90% of particles have a size of 200-600 nm. This size was selected for the experiments as it is corresponds to the wavelength of UV light or visible light, which plays an important role in white appearance of teeth. In the context of the invention, "about" means +/- 10 %, preferably, +/- 5%.

In one embodiment, the particles have a mixture of different sizes, which may provide a still more intense whitening effect. In particular, for example 30-70 % of particles may have a size of 200-400 nm, 30-70 % of particles have a size of 400-600 nm, and, optionally, 30-70% of particles have a size of 10-200 nm.

The dental care product is selected from the group comprising toothpaste, prophylactic paste, tooth powder, tooth polish, tooth gel, chewing gum, candy, lozenge, mouthwash, whitening strip, varnish, veneer, and tube, syringe or dental tray comprising a gel or paste. Preferably, the dental care product is a toothpaste or a tooth gel comprising 0.5-40 wt% of said mineral particles and 0.5-1 wt% of said organic matrix, preferably, protein matrix.

The dental care product may additionally comprise one or more typical ingredients of the respective dental care product. Such typical ingredients may be:
- abrasive agents such as carbonates, phosphates, silicates, acrylates, alumina,
- suspension agents such as glycerine, polyethylene glycols (PEG), sorbitol, xylitol,
- binding agents such as cellulose and derivate thereof, carrageenan, paraffin, xylose,
- detergents such as hydrogenated castor oil, sodium lauryl sulphate,
- aroma such as caramel, vanillin, menthol,
- conserving agent such as ethanol, sodium benzoate,
- colouring agents such as solvent red, acid blue 3,
- active agents such as fluorides, preferably, in the form of tertiary amines, such as amine fluoride or organic fluoride such as sodium monofluorophosphat, potassium nitrate, and/or oxalate.

In one embodiment of the invention, the product is a toothpaste comprising all or the main ingredients of Curodont™ Repair (i.e., Oligopeptide 104 (SEQ ID NO: 1) and a bulking agent) or, preferably, of Curodont™ Protect, (both available from credentis ag, Switzerland) and added mineral particles, in particular, calcium phosphate, preferably, hydroxyapatite particles and/or another crystalline form, most preferably, hydroxyapatite. Thus, ingredients may be, e.g., hydroxyapatite particles and/or calcium phosphate in another crystalline form,, preferably, hydroxyapatite, hydrogenated starch hydrolysate, aqua, hydrated silica, PEG-8; cellulose gum, sodium monofluorophosphate, aroma, sodium saccharin, citric acid, sodium hydroxyde, dicalcium phosphate, oligopeptide-104, calcium glycerophosphate, sodium chloride, sodium sulfate, limo-nene, cinnamal, and CI 42090.

The present invention also relates to cosmetic use of the dental care product of the invention for tooth whitening. A method of tooth whitening for cosmetic reasons is also disclosed, comprising administering the dental care product of the invention to a tooth. In the context of the invention, unless explicitly mentioned or clear from the context, "a" is not limited to the singular, but can also mean "one or more". For example, reference to "a tooth" includes the option that more than one tooth, in particular, all teeth of one person, are designated. The dental care product of the invention may also be used for whitening crowns, implants, filling materials and other oral appliances.

In the method of the invention, the composition is preferably administered one, two or three times a day on 1, 2, 3, 4, 5, 6, 7 or more days, in one embodiment, daily. It can also be administered less often, e.g., once a week or once a month. Frequency of administration is strongly dependent on the whitening effect desired by the user, as well as on the amount of mechanical abrasion to which the teeth are subjected. This includes life-long administration, preferably starting after the permanent teeth have erupted, in particular, after discoloration of a tooth has been noticed. Administration means that a tooth, or, preferably, all teeth of a person are contacted with the dental care product in the way this respective type of product is typically used. For example, a toothpaste is typically used to brush teeth for a time of 1-5 minutes, in particular, about 2-3 minutes.

As the dental care product of the invention does not lead to the undesired side effects of bleaching products comprising peroxides, it can be used daily for all cycles of dental care without harm to the teeth or the gums. As the dental care product may further enhance remineralisation of teeth, reduce tooth sensitivity and prevents caries, and may even be used to treat beginning caries lesions, no additional dental care product is required. In particular, the dentifrice or toothpaste of the invention may be used for every brushing of teeth. Alternatively, it may be used in addition to or alternately with a different dentifrice, e.g., an alternative fluoride containing toothpaste. For example, an alternative fluoride containing toothpaste may be used in the mornings, and the toothpaste of the invention may be used in the evening after the last meal of the day.

The present invention also provides the dental care product of the invention for use in treatment of a sensitive tooth and/or for prevention or treatment of caries. Also disclosed is a method of treating a sensitive tooth and/or of preventing and /or treating caries and/or tooth whitening comprising administrating an effective amount of the dental care product of the invention to a tooth or to teeth.

The following examples are intended to illustrate, but not to limit the invention. All literature cited in the present application is herewith incorporated herein in full.

### Legends:

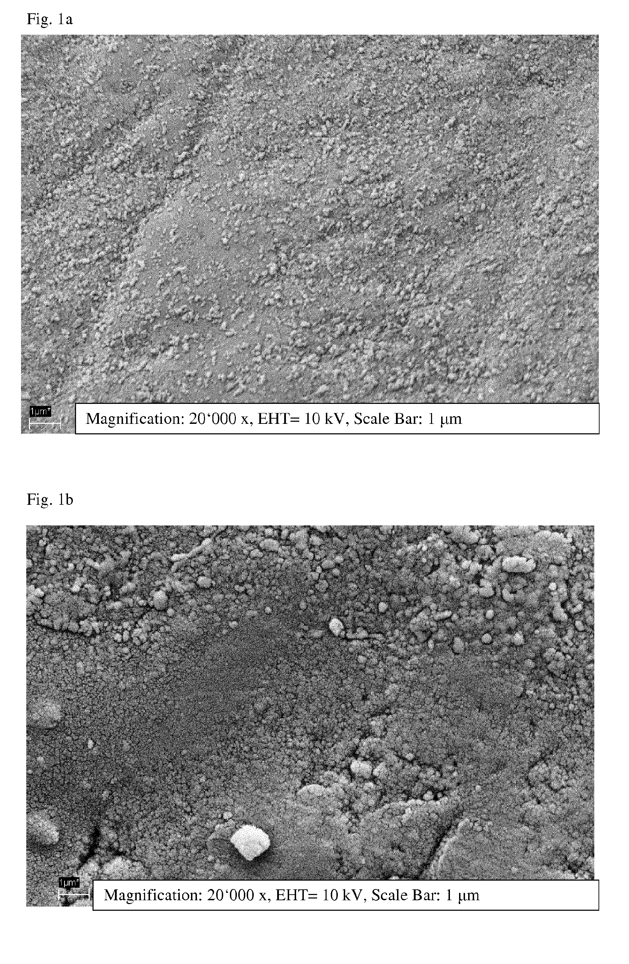

Fig. 1 (A) Electron microscopy picture of toothsurface treated with mixture of P11-4 matrix and hydroxyapatite particle suspension. The surface of the tooth shows tightly bound particles. (B) Electron microscopy picture of tooth surface treated only with hydroxyapatite particle suspension in water. The surface shows unregular positioning of particles on the surface.

### Examples

### Materials and Methods

Suspensions of oligopeptide 104 (10mg/ml) with or without hydroxyapatite particles (average size 400nm; 40-60% crystallinity) were generated.

The suspensions were directly applied onto an enamel surface of a tooth, and residues washed off (10 sec). The specimen was stored in distilled water for 24 hours at 37°C. The procedure was repeated 3 times.

The tooth colour was measured with a dental spectrophotometer (VITA Easyshade). The illumination conditions were standardized with a black box as the background for the teeth during the measurement. The tip was applied perpendicular to the tooth surface and the average L*a*b-values from three repetitons were used for evaluations. Colour measurement were done at baseline (t1= without treatment), 24 hours after first application (t1), 24 hours after second application (t3), 24 hours after the third application (t4).

The mean changes of the L*a*b-values between different measurements in each group were expressed as ΔE (according to ISO 28399).

### Results

The results are provided in Table 2 below:

**Table 2**

| | Control (HA only) | Test (HA + oligopeptide 104) |
|---|---|---|
| ΔE (t2-t1) - 1^{st} application | 2.3 | 4.6 |
| ΔE (t3-t2) - 2^{nd} application | 1.1 | 1.9 |
| ΔE (t4-t3) - 3^{rd} application | 1.5 | 0.6 |

The experiment shows that, surprisingly, the combination of a protein matrix according to the invention with HA significantly increases the whitening effect seen upon application of HA only.

### Literature

Dabanoglu et al., 2009, Am J Dent 22:23-29.
Dahl et al., 2003, Crit Rev Oral Biol Med 14(4):292-304.
Jiang et al, 2008, J Dent 36(11): 907-914.
Jin et al., 2013, Eur I Oral Sci 121: 382-388.
Lim et al., 2009, Biomed Mater 4(2): 025017.
Mohd et al., 2007, Biomed Mater Eng 17(2): 69-75.
Niwa et al. J Mater Sci Mater Med 2001; 12: 277-281.
Raoufi, S. and D. Birkhed (2010). Int Dent J60(6): 419-423.
Roveri, Battistelli et al., 2009, J Nanomaterial, special issue, Article ID 746383
EP 1 762 215 A1, EP 2 327 428 A2
US 20050037948 A1, US 20080075675 A1, US 2008199431 A1, US 20100247589 A1, US 20100297203 A1
US 6,548,630
WO 2004/007532 A1, WO 2006/073889 A2, WO 2007/000979 A1, WO 2006/047315 A2, WO 2007/137606 A1, WO 2008/113030 A2, WO 2009/026729 A1, WO 2010/041636 A1, WO 2010/103887 A1, WO 2013/068020 A1

## Claims

1. Dental care product comprising 0.5-40 wt% mineral particles, the particles having a size of 10-1200 nm, and 0.01-5 wt% of a protein capable of forming a protein matrix which is a hydrogel, wherein the dental care product comprises a fluorophore.

2. Dental care product according to claim 1, wherein said fluorophore is an amino acid residue of the protein, preferably Trp, wherein preferably 5% or more of the residues of the protein are Trp.

3. Dental care product according to any of the preceding claims, wherein the protein forms a hydro gel.

4. Dental care product according to any of the preceding claims, wherein said protein is selected from the group comprising collagen, amelogenin, serum albumin, lysozyme, a self-assembling peptide, and a supramolecular assembly.

5. Dental care product according to any of the preceding claims, wherein said protein is a self-assembling peptide having at least 60% sequence identity to SEQ ID NO: 1 or SEQ ID NO:2, or preferably, is a self-assembling peptide comprising SEQ ID NO: 1 or SEQ ID NO: 2.

6. Dental care product according to any of the preceding claims, wherein said mineral particles are calcium phosphate particles, calcium carbonate particles, kaolin, or titanium oxide particles, preferably, calcium phosphate particles.

7. Dental care product according to any of the preceding claims, wherein said mineral particles comprise hydroxyapatite.

8. Dental care product according to any of the preceding claims, wherein the dental care product is selected from the group comprising toothpaste, prophylactic paste, tooth powder, tooth polish, tooth gel, chewing gum, candy, lozenge, mouthwash, whitening strip, varnish, veneer, and tube, syringe or dental tray comprising a gel or paste.

9. Dental care product according to any of the preceding claims, comprising 0.5-40 wt% of said mineral particles and 0.5-1 wt% of said matrix protein, wherein the dental care product is a toothpaste or gel.

10. Dental care product according to any of the preceding claims, wherein 30-70 % of said mineral particles have a size of 200-600 nm.

11. Dental care product according to any of the preceding claims, wherein the product comprises Curodont™ Protect or Curodont™ Repair and added hydroxyapatite particles.

12. Cosmetic use of the dental care product of any of the preceding claims for tooth whitening.

13. The dental care product of any of claims 1-11 for use in treatment of a sensitive tooth and/or for prevention or treatment of caries.

14. Cosmetic method of tooth whitening, comprising administering the dental care product of any of claims 1-11 to a tooth.

15. Cosmetic method of claim 14, wherein the composition is administered one, two or three times a day on 1, 2, 3, 4, 5, 6, 7 or more days, preferably, daily.
